# EUROPÄISCHE PATENTSCHRIFT

(11) **EP 1 370 509 B1**
(45) Veröffentlichungstag und Bekanntmachung des Hinweises auf die Patenterteilung: **26.05.2010**
(21) Anmeldenummer: 02726140.3
(22) Anmeldetag: 05.03.2002
(51) Int. Cl.: C07C 51/06, C07C 51/09, C07C 51/41

(54) **VERFAHREN ZUR HERSTELLUNG VON GLYCERINSÄUREN**
METHOD FOR THE PRODUCTION OF GLYCERIC ACID
PROCEDE DE PRODUCTION D'ACIDES GLYCERIQUES

(30) Priorität: 07.03.2001 DE 10110849
(43) Veröffentlichungstag der Anmeldung: 17.12.2003
(73) Patentinhaber: BASF SE, 67056 Ludwigshafen (DE)
(72) Erfinder: GROSCH, Georg, Heinrich, 67098 Bad Dürkheim (DE); BOCHNITSCHEK, Werner, 67067 Ludwigshafen (DE); NEUMANN, Peter, 68309 Mannheim (DE); KINGMA, Arend, Jouke, 67069 Ludwigshafen (DE)
(86) Internationale Anmeldenummer: PCT/EP2002/002406
(87) Internationale Veröffentlichungsnummer: WO 2002/070450

(56) Entgegenhaltungen:
- GB-A- 1 286 894
- SUGIYAMA, S ET AL: "Selective preparation of 2,3-epoxypropanamide and its facile conversion to 2,3-dihydroxypropanamide with acidic resins" BULLETIN OF THE CHEMICAL SOCIETY OF JAPAN., Bd. 62, Nr. 10, 1989, Seiten 3202-3206, XP002206282 JAPAN PUBLICATIONS TRADING CO. TOKYO., JP ISSN: 0009-2673
- ENGLISH J ET AL: "The dehydration of some alpha,beta-dihydroxy esters" JOURNAL OF THE AMERICAN CHEMICAL SOCIETY., Bd. 77, 1955, Seiten 4661-4664, XP002206283 AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 356606 XP002206284 & ARTAMONOW: ZHURNAL OBSHCHEI KHIMII., ENGL AUSG., Bd. 28, 1958, Seiten 1414-1417, NAUKA, MOSCOW., RU ISSN: 0044-460X
- DATABASE CROSSFIRE BEILSTEIN [Online] Beilstein Institut zur Förderung der Chemischen Wissenschaften, Frankfurt am Main, DE; Database accession no. 329484 XP002206285 & BRADFIELD ET AL: JOURNAL OF THE CHEMICAL SOCIETY., 1937, Seiten 760-763, CHEMICAL SOCIETY, LONDON, GB ISSN: 0368-1769
- DATABASE CROSSFIRE BEILSTEIN [Online] Beilstein Institut zur Förderung der Chemischen Wissenschaften, Frankfurt am Main, DE; Database accession no. 282014 XP002206286 & MURRAY ET AL: JOURNAL OF THE AMERICAN CHEMICAL SOCIETY., Bd. 56, 1934, Seite 2749 AMERICAN CHEMICAL SOCIETY, WASHINGTON, DC., US ISSN: 0002-7863
- PATENT ABSTRACTS OF JAPAN vol. 1998, no. 10, 31. August 1998 (1998-08-31) & JP 10 127299 A (NITTO CHEM IND CO LTD;MITSUBISHI RAYON CO LTD), 19. Mai 1998 (1998-05-19)

## Beschreibung

Die vorliegende Erfindung betrifft Verfahren zur Herstellung von, gegebenenfalls substituierter, Glycerinsäure.

Glycerinsäure ist ein für chemische Synthesen interessanter Chemikalienbaustein und ein Zwischenprodukt für physiologisch aktive Verbindungen und Aminosäuren.

Unterschiedliche Verfahren zur Herstellung von Glycerinsäure sind bekannt.

In der DE-A 42 28 487 ist die Herstellung von Glycerinsäure durch Oxidation von Glycerin mit Sauerstoff in Gegenwart von Katalysatoren beschrieben. Es wird insbesondere ein Katalysator eingesetzt, der 1% Ce und 5% Pt auf Aktivkohle enthält, siehe Beispiel 13.

JP-A 60 226 842 betrifft die Herstellung von Glycerinsäure durch Umsetzung von Acrylsäure mit Wasserstoffperoxid in Gegenwart eines Wolfram enthaltenden Katalysators.

US 3,846,478 betrifft die Oxidation von olefinischen Verbindungen zur Bildung von Glykolen. Dabei kann Glycerinsäure ebenfalls durch katalytische Oxidation von Acrylsäure hergestellt werden. Weiterhin ist die Oxidation von Acrylamid zu Glycerinsäureamid in Beispiel 14 beschrieben. Die Oxidation wird in Gegenwart von Osmiumtetroxid mit einem Alkalihypochlorit oder Erdalkalihypochlorit durchgeführt.

J. English et al., J. Am. Chem. Soc., Band 77,1955, Seiten 4661 bis 4664 betrifft die Herstellung und Entwässerung von α,β-Hydroxyestern. Auf Seite 4662 ist die Ringöffnung eines entsprechenden *erythro*-Ethyl-α,β-dehydroxycaproat zu der entsprechende Carbonsäure durch Behandlung mit Kalilauge beschrieben. Der als Ausgangsstoff verwendete Ester wird durch eine Darzens-Glycidesterkondensation aus Butyralaldehyd und Ethylchloroacetat hergestellt.

Die ringöffnende Addition von Wasser an den Epoxidring erfolgt an einem Capronsäureester.

Das Beilstein-Referat *Reaction ID 329484* bezieht sich auf eine Epoxid-Ringöffnung durch Umsetzung mit wässriger Schwefelsäure. Das Beilstein-Referat beschreibt aber nicht, wie der entsprechende Ester synthetisiert wird. Insbesondere fehlt die Information, wie die Epoxidfunktion erzeugt wird (Glycidestersynthese nach Darzens, Epoxidierung etc.). Das Beilstein-Referat beschreibt Ethylester als Ausgangsprodukt.

S. Sugijama et al., Bull. Chem. Soc. Jpn., 62, 1989, Seiten 3202 bis 3206 offenbart ein Verfahren zur Herstellung von 2,3-Dihydroxypropanamid durch Umsetzung von Acrylnitril mit Wasserstoffperoxid und anschließender säurekatalysierter ringöffnender Addition von Wasser an den Epoxidring. Beim Verfahren zur Epoxidierung von Glycidsäureamiden mit anschließender Öffnung des Epoxids wird allerdings die Carbonsäureamid-Funktion im zweiten Verfahrensschritt der Epoxid-Ringöffnung nicht verseift. Unter den angegebenen Verfahrensbedingungen der Epoxidöffnung (verdünnte alkalische Lösung) ist das Carbonsäureamid stabil.

Nachteilig bei den unterschiedlichen Verfahrensvarianten sind eine zu geringe Selektivität bei der Oxidation von Glycerin und eine sehr aufwendige Abtrennung der Nebenkomponenten vom gewünschten Produkt. Zudem ist die Abtrennung von homogenen Katalysatoren vom Zielprodukt bei der Acrylsäureoxidation problematisch.

Aufgabe der vorliegenden Erfindung ist die Bereitstellung eines Verfahrens zur Herstellung von, gegebenenfalls substituierter, Glycerinsäure, das mit hoher Selektivität zur, gegebenenfalls substituierten, Glycerinsäure führt und eine einfache Abtrennung von Nebenkomponenten erlaubt.

Die Aufgabe wird erfindungsgemäß gelöst durch ein Verfahren zur Herstellung von Glycerinsäureverbindungen der allgemeinen Formel (I)

R¹R²C(OH)-CR³(OH)-COOX (I)

in der R¹, R² und R³ unabhängig Wasserstoff, C₁₋₁₂-Alkyl, C₆₋₁₂-Aryl, C₇₋₁₃-Alkaryl oder C₇₋₁₃-Aralkyl und
X Wasserstoff, Na oder NH₄ bedeuten,
durch basenkatalysierte Verseifung von Glycidsäureverbindungen der allgemeinen Formel (II) 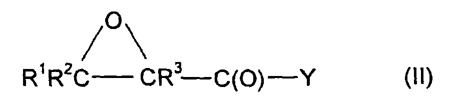 in der Y NH₂ bedeutet,
mit einer 10 bis 50 gew.-% igen Natronlauge
unter ringöffnender Addition von Wasser an den Epoxidring mit den Merkmalen von Anspruch 1.

Es wurde erfindungsgemäß gefunden, daß Glycidsäureamide oder Glycidsäureester durch Verseifung unter ringöffnender Addition von Wasser an den Epoxidring in hohen Ausbeuten und Selektivitäten zu den gewünschten, gegebenenfalls substituierten, Glycerinsäuren umgesetzt werden können.

In den allgemeinen Formeln (I) und (II) bedeuten R¹, R² und R³ vorzugsweise unabhängig Wasserstoff, C₁₋₆-Alkyl, Phenyl, C₇₋₁₀-Alkylphenyl oder C₇₋₁₀-Phenylalkyl. Besonders bevorzugt sind R¹, R² und R³ unabhängig Wasserstoff oder C₁₋₆-Alkyl, insbesondere Wasserstoff oder C₁₋₃-Alkyl. Besonders bevorzugt sind R¹, R² und R³ Wasserstoff, so daß es sich bei der Verbindung der allgemeinen Formel (I) um Glycerinsäure oder deren Salze und bei der Verbindung der allgemeinen Formel (II) um Glycidsäureamid handelt. Insbesondere bedeuten R¹, R² und R³ Wasserstoff und Y NH₂, so daß es sich bei der Glycidsäureverbindung der allgemeinen Formel (II) um Glycidsäureamid handelt.

Die Herstellung von Glycidsäureamid und Glycidsäureestern ist an sich bekannt. Entsprechende Verfahren sind beispielsweise in DE-A 19 04 077, DE-A 37 12 330 und DE-A 38 29 829 beschrieben.

Dabei bedeutet Y NH₂, und das eingesetzte Glycidsäureamid der allgemeinen Formel (II) wird durch Umsetzung von Acrylnitrilen der allgemeinen Formel (III)

R¹R²C = CR³CN (III)

mit Wasserstoffperoxid hergestellt. Die Herstellung von Glycidsäurenitril durch Umsetzung von Acrylnitril mit Wasserstoffperoxid ist an sich bekannt und beispielsweise in DE-A 19 04 077 und DE-A 38 29 829 beschrieben. Für entsprechende Herstellungsverfahren kann auf diese Schriften verwiesen werden. Als mögliche Einsatzgebiete von Glycidsäureamid werden in diesen Schriften die Herstellung von Textilhilfsmitteln, Pflanzenschutzmitteln, Konservierungsmitteln sowie die Herstellung von Farbstoffen oder die Herstellung von Komplexbildnern wie Isoserin-N,N-diessigsäure genannt, nicht jedoch die Herstellung von Glycerinsäure. Auch die DE-A 37 12 330 beschreibt die Verwendung von Glycidsäureamid zur Herstellung von Komplexbildnern.

Die Kombination der Umsetzung von Acrylnitrilen mit Wasserstoffperoxid zu Glycerinsäureamiden und die nachfolgende Verseifung unter ringöffnender Addition von Wasser an den Epoxidring führt in einem unaufwendigen Gesamtverfahren mit hohen Ausbeuten und Selektivitäten zu der gewünschten, gegebenenfalls substituierten, Glycerinsäure, wobei im Reaktionsgemisch verbleibende Ausgangsstoffe oder Nebenprodukte einfach abgetrennt werden können. Zudem kann auf den Einsatz von Katalysatoren, wie sie im Stand der Technik beschrieben sind, verzichtet werden.

Die Verseifung wird basenkatalysiert durchgeführt.

Nach der Umsetzung der Acrylnitrile der allgemeinen Formel (III) können nicht umgesetzte Acrylnitrile der allgemeinen Formel (III) und andere Nebenprodukte destillativ vom Reaktionsgemisch abgetrennt werden.

Nach der Umsetzung der Acrylnitrile der allgemeinen Formel (III) mit Wasserstoffperoxid kann zudem nicht umgesetztes Wasserstoffperoxid zersetzt werden.

Zudem kann der bei der Verseifung entstehende Ammoniak abdestilliert werden.

Nach der basisch katalysierten Verseifung entstehende Glycerinsäuresalze der allgemeinen Formel (I) können in freie Glycerinsäuren überführt werden.

Am Beispiel von Glycerinsäure kann das Gesamtverfahren wie folgt zusammengefaßt werden:
a) Umsetzung von Acrylnitrilen mit wäßrigem Wasserstoffperoxid zu Glycidsäureamid,
b) gegebenenfalls destillative Entfernung von nicht umgesetztem Acrylnitril und anderen Nebenprodukten aus Schritt a),
c) gegebenenfalls Zersetzung des nicht umgesetzten Wasserstoffperoxids aus Schritt a),
d) Verseifung des erhaltenen Glycidsäureamids zu einem Alkali- oder Erdalkalisalz der Glycerinsäure oder zu Glycerinsäure,
e) gegebenenfalls Abdestillieren des entstehenden Ammoniaks,
f) gegebenenfalls Umwandlung der Natriumsalze der Glycerinsäure in (freie) Glycerinsäure.

Das erfindungsgemäße Verfahren kann kontinuierlich oder diskontinuierlich durchgeführt werden. Die Verfahrensschritte (a) und (d) werden vorzugsweise in einer Rührkesselkaskade durchgeführt. Es sind aber auch andere Ausführungsformen möglich. So können die Rührkessel teilweise oder ganz gegen Rohrreaktoren ausgetauscht werden.

Die Einzelschritte (a) bis (f) werden nachfolgend anhand teilweise bevorzugter Ausführungsformen und am Beispiel der nicht substituierten Glycerinsäure näher erläutert:
(a) Acrylnitril und eine wäßrige Wasserstoffperoxidlösung, deren Gehalt im Bereich von 3 bis 50 Gew.-%, vorzugsweise 10 bis 25 Gew.-% liegt, werden im molaren Verhältnis 1:0,6 bis 1:1,5, vorzugsweise 1:1 bis 1:1,2 in einem Rührkessel bei einem pH-Wert von 7 bis 8, vorzugsweise 7,3 bis 7,7 und einer Temperatur von 30°C bis 60°C, vorzugsweise 45°C bis 55°C miteinander zur Reaktion gebracht. Die mittlere Verweilzeit im Rührkessel beträgt üblicherweise 30 bis 60 Minuten.
   Zur Konstanthaltung des pH-Wertes und der Temperatur wird 5 bis 50 gew.-%ige, vorzugsweise 10-20 gew.-%ige Natronlauge zudosiert. In einem bis drei, vorzugsweise einem nachgeschalteten Rührkessel wird bei den gleichen oder ähnlichen Bedingungen bezüglich pH-Wert und Temperatur die Reaktionslösung nachvermischt. Die mittlere Verweilzeit im zweiten Rührkessel beträgt üblicherweise 15 bis 90 Minuten, bevorzugt 15 bis 45 Minuten.
(b) Das bei (a) nicht umgesetzte Acrylnitril, dessen Anteil normalerweise 5 bis 50 Gew.-%, vorzugsweise 10 bis 30 Gew.-% der eingesetzten Menge beträgt, sowie mögliche Nebenkomponenten in Schritt a), die niedrigere Siedepunkte als Glycidamid haben, werden in einer Destillationskolonne, die vorzugsweise nach dem Dünnschichtverdampfungsprinzip arbeitet, abgetrennt. Die Destillationskolonne wird vorzugsweise bei der gleichen Temperatur wie in Schritt a) und bei einem Druck von vorteilhafterweise von 50 bis 160 mbar betrieben. Im Destillat befindet sich ferner Wasser.
   Der Restgehalt an Acrylnitril nach der Destillation in der Reaktionslösung beträgt zwischen 0,1 und 4 Gew.-%. Das abgetrennte Acrylnitril kann gegebenenfalls nach Abtrennung von weiteren Stoffen in den Schritt a) zurückgeführt werden.
(c) Schritt (c) ist optional. Je nachdem, wie die Verseifung des Glycidsäureamids durchgeführt wird, ist die Zersetzung des überschüssigen Wasserstoffperoxids vor der Verseifung des Glycidsäureamids notwendig oder nicht. Die Reaktionslösung wird zur Zersetzung von noch vorhandenem Wasserstoffperoxid über einen festen Kontakt, vorzugsweise aus Aktivkohle, geleitet. Es können aber auch andere feste Materialien zur Zerstörung des Wasserstoffperoxids, beispielsweise Zeolithe oder wasserunlösliche Mangan-, Blei-, Vanadium- oder Edelmetallverbindungen, verwendet werden. Die Reihenfolge der Schritte (b) und (c) kann vertauscht werden.
(d) Die Verseifung des Glycidsäureamids erfolgt basisch.
   Bei der basischen Verseifung wird die Glycidamidlösung mit einer 10 bis 50 gew.-%igen, vorzugsweise 40 bis 50 gew.-%igen Lösung von Natronlauge , im molaren Verhältnis von 1:1 bis 1:1,7, vorzugsweise 1;1,3 bis 1;1,4 in einem Rührkessel bei einer Temperatur von 60 bis 100°C, vorzugsweise 70 bis 95°C und einem pH-Wert von 9 bis 14, vorzugsweise 11 bis 12,5 zum Endprodukt verseift. Der größte Teil des durch die Verseifung entstehenden Ammoniaks kann schon während der Verseifung durch Anlegen eines verminderten Druckes von 200 bis 700 mbar oder durch Strippen mit Stickstoff oder einem anderen Inertgas aus dem Produkt entfernt werden.
   Optional kann zu Beginn von Schritt (d) vor der eigentlichen Verseifungsreaktion eine thermische Behandlung der aus Schritt (c) gewonnenen wäßrigen Glycidsäureamidlösung bei Temperaturen von 50 bis 100°C und einer Dauer von 10 min bis 3 h erfolgen. Optional kann die Konzentration der aus Schritt (c) gewonnenen Glycidsäureamidlösung durch Aufkonzentrieren oder Verdünnen mit z.B. Wasser verändert werden. Ferner können optional saure oder basische, homogene oder heterogene Katalysatoren zugesetzt werden.
   Eine spezielle Ausführungsform des Verfahrens sieht vor, dass die Glycidsäureamidlösung vor der Verseifung mit oder ohne CO₂ in Gegenwart eines Katalysators behandelt wird. Solche Katalysatoren können z. B. Metallhalogenide, Tetraalkylammonium- oder -phosphoniumhalogenide sowie Metalloxide, Metallhydrogencarbonate, Metallcarbonate, Metallhydroxide und Metallate wie Molybdat, Vanadat, Wolframat sein. Die Behandlung der Glycidsäureamidlösung mit CO₂ erfolgt bei Temperaturen von vorzugsweise 50°C bis 180°C und Drücken von vorzugsweise 1 bar bis 30 bar. Anschließend wird die so behandelte Glycidsäureamidlösung der Verseifung zugeführt.
(e) Die restliche Menge des bei (d) bei der basischen Verseifung entstandenen Ammoniaks wird als Mischung mit Wasser in einer Stripp- oder Destillationskolonne, die vorzugsweise nach dem Dünnschichtverdampferprinzip arbeitet, abgetrennt. Die Destillationskolonne wird vorzugsweise bei gleicher Temperatur wie bei Schritt (d) und bei einem Druck von 200 bis 700 mbar, vorzugsweise 400 bis 600 mbar, betrieben. Es verbleibt eine in der Regel 2 bis 20 gew.-%ige Lösung des Glycerinsäuresalzes, welche durch Abdestillieren von Wasser aufkonzentriert werden kann.
(f) Ist die Zielverbindung die Glycerinsäure, so schließt sich an Schritt (e) die Umwandlung des Glycerinsäuresalzes in die Glycerinsäure an.

Die Vorteile des erfindungsgemäßen Verfahren sind:
- Selektivität zu Glycidamid bei dessen Herstellung aus Acrylnitril und Wasserstoffperoxid von > 60%, bezogen auf das umgesetzte Acrylnitril.
- Einfache Abtrennung der nicht umgesetzten Edukte, sowie gebenenfalls Abtrennung von unerwünschten Nebenprodukten.
- Die Verseifung von Glycidamid zur Glycerinsäure verläuft praktisch quantitativ.

Die Erfindung wird durch die nachstehenden Beispiele näher erläutert:

### Beispiele:

### Beispiel 1: Herstellung von Glycidsäureamid aus Acrylnitril

a) Pro Stunde werden 76,4 (1,44 mol) Acrylnitril (AN) und 343 g einer 15 gew.-%igen wäßrigen Wasserstoffperoxidlösung (entsprechen 1,51 mol H₂O₂) zusammen mit stündlich 27 g einer 8 gew.-%igen Natronlauge bei 50°C in einen Rührkessel R1 (Volumen 0,3 1) so eindosiert, daß der pH-Wert zwischen 7,4 und 7,5 gehalten wurde. Die Reaktionsmischung gelangte nach einer Verweilzeit von 40 min in den Rührkessel R2 (Volumen 0,5 1), wo weitere 29 g 8 gew.-%iger Natronlauge pro Stunde zur Konstanthaltung des genannten pH-Wertes eingetragen wurden. Die Temperatur im Rührkessel R2 betrug 50°C, die Verweilzeit 63 min.
b) Aus der Reaktionsmischung wurden im Sambay-Dünnschichtverdampfer D1, der bei 50°C und 70 mbar betrieben wurde, stündlich 90 g Destillat, bestehend aus nicht umgesetztem Acrylnitril und Wasser abdestilliert. Als Sumpf wurden stündlich 385 g einer wäßrigen Glycidsäureamidlösung erhalten, die gemäß Gaschromatograph einen Gehalt an Glycidsäureamid von ca. 60 Flächenprozent hatte.

### Beispiel 2: Ringöffnende Verseifung von Glycidsäureamid zu Natriumglycerat

250g einer Glycidsäureamidlösung aus Beispiel 1 werden mit 613 g einer 18,5 gew.-%igen Natronlauge bei einer Temperatur von 100°C und einem pH-Wert von 11,0 bis 11,5 zu Natriumglycerat verseift.

## Patentansprüche

1. Verfahren zur Herstellung von Glycerinsäureverbindungen der allgemeinen Formel (I)
R¹R²C(OH)-CR³(OH)-COOX (I)
in der R¹, R² und R³ unabhängig Wasserstoff, C₁₋₁₂-Alkyl, C₆₋₁₂-Aryl, C₇₋₁₃-Alkaryl oder C₇₋₁₃-Aralkyl und
X Wasserstoff, Na oder NH₄ bedeuten,
durch basenkatalysierte Verseifung von Glycidsäureverbindungen der allgemeinen Formel (II) 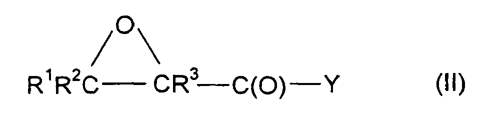 in der Y NH₂ bedeutet,
mit einer 10 bis 50 Gew.-%-igen Natronlauge
unter ringöffnender Addition von Wasser an den Epoxidring, wobei das eingesetzte Glycidsäureamid der allgemeinen Formel (II) durch Umsetzung von Acrylnitrilen der allgemeinen Formel (III)
R¹R²C=CR³CN (III)
mit Wasserstoffperoxid hergestellt wird, bei einem pH-Wert von 7 bis 8 und einer Temperatur von 30 bis 60°C, wobei zur Konstanthaltung des pH-Werts und der Temperatur 5 bis 50 Gew.-%-ige Natronlauge zudosiert wird.

2. Verfahren nach Anspruch 1, **dadurch gekennzeichnet, dass** R¹, R² und R³ unabhängig Wasserstoff oder C₁₋₆-Alkyl bedeuten.

3. Verfahren nach Anspruch 1 oder 2, **dadurch gekennzeichnet, dass** R¹, R² und R³ Wasserstoff bedeuten.

4. Verfahren nach einem der Ansprüche 1 bis 3, **dadurch gekennzeichnet, daß** nach der Umsetzung der Acrylnitrile der allgemeinen Formel (III) mit Wasserstoffperoxid nicht umgesetzte Acrylnitrile der allgemeinen Formel (III) und andere Nebenprodukte destillativ vom Reaktionsgemisch abgetrennt werden.

5. Verfahren nach Anspruch einem der Ansprüche 1 bis 4, **dadurch gekennzeichnet, daß** nach der Umsetzung der Acrylnitrile der allgemeinen Formel (III) mit Wasserstoffperoxid nicht umgesetztes Wasserstoffperoxid zersetzt wird.

6. Verfahren nach einem der Ansprüche 1 bis 5, **dadurch gekennzeichnet, dass** der bei der Verseifung entstehende Ammoniak abdestilliert wird.

7. Verfahren nach einem der Ansprüche 1 bis 6, **dadurch gekennzeichnet, dass** nach der basisch katalysierten Verseifung entstehende Glycerinsäuresalze der allgemeinen Formel (I) in freie Glycerinsäuren überführt werden.

## Claims

1. A process for preparing glyceric acid compounds of the formula (I)
R¹R²C (OB) -CR³ (OB) -COOX (I)
where R¹, R² and R³ are independently hydrogen, C₁₋₁₂-alkyl, C₆₋₁₂-aryl, C₇₋₁₃-alkaryl or C₇₋₁₃-aralkyl and
X is hydrogen, Na or NH₄,
by base-catalyzed saponification of glycidic acid compounds of the formula (II) 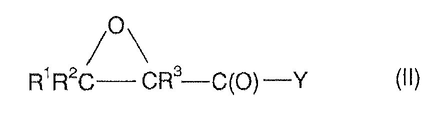 where Y is NH₂,
with a 10 to 50% strength by weight aqueous sodium hydroxide solution
with ring-opening addition of water onto the epoxide ring, where the glycidamide of the formula (II) which is used is prepared by reacting acrylonitriles of the formula (III)
R¹R²C=CR³CN (III)
with hydrogen peroxide, at a pH of from 7 to 8 and a temperature of from 30 to 60°C, with 5 to 50% strength by weight aqueous sodium hydroxide being metered in to keep the pH and the temperature constant.

2. The process according to claim 1, wherein R¹, R² and R³ are independently hydrogen or C₁₋₆-alkyl.

3. The process according to claim 1 or 2, wherein R¹, R² and R³ are hydrogen.

4. The process according to any of claims 1 to 3, wherein unreacted acrylonitriles of the formula (III) and other by-products are separated from the reaction mixture by distillation after the reaction of the acrylonitriles of the formula (III) with hydrogen peroxide.

5. The process according to any of claims 1 to 4, wherein unreacted hydrogen peroxide is decomposed after the reaction of the acrylonitriles of the formula (III) with hydrogen peroxide.

6. The process according to any of claims 1 to 5, wherein the ammonia formed in the saponification is distilled off.

7. The process according to any of claims 1 to 6, wherein glyceric acid salts of the formula (I) formed in the base-catalyzed saponification are converted into free glyceric acids.

## Revendications

1. Procédé pour la préparation de composés de l'acide glycérique de formule générale (I),
R¹R²C (OH) -CR³ (OH ) -COOX (I)
dans laquelle
R¹, R² et R³ signifient, indépendamment, hydrogène, C₁₋₁₂-alkyle, C₆₋₁₂-aryle, C₇₋₁₃-alkaryle ou C₇₋₁₃-aralkyle et
X signifie hydrogène, Na ou NH₄,
par saponification catalysée par des bases de composés de l'acide glycidique de formule générale (II) 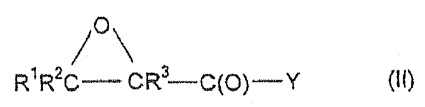 dans laquelle Y signifie NH₂
avec une lessive de soude caustique à 10 jusqu'à 50% en poids
sous addition avec ouverture de cycle d'eau sur le cycle époxyde, l'amide de l'acide glycidique utilisé de formule générale (II) étant préparé par transformation d'acrylonitriles de formule générale (III)
R¹R²C=CR³CN (III)
avec du peroxyde d'hydrogène à un pH de 7 à 8 et une température de 30 à 60°C, en ajoutant en dosant, pour conserver le pH constant et la température constante, de la lessive de soude caustique de 5 à 50% en poids.

2. Procédé selon la revendication 1, **caractérisé en ce que** R¹, R² et R³ signifient, indépendamment, hydrogène ou C₁₋₆-alkyle.

3. Procédé selon la revendication 1 ou 2, **caractérisé en ce que** R¹, R² et R³ signifient hydrogène.

4. Procédé selon l'une quelconque des revendications 1 à 3, **caractérisé en ce qu'**après la transformation des acrylonitriles de formule générale (III) avec du peroxyde d'hydrogène, les acrylonitriles non transformés de formule générale (III) et d'autres produits secondaires sont séparés par distillation du mélange réactionnel.

5. Procédé selon l'une quelconque des revendications 1 à 4, **caractérisé en ce qu'**après la transformation des acrylonitriles de formule générale (III) avec du peroxyde d'hydrogène, le peroxyde d'hydrogène non transformé est décomposé.

6. Procédé selon l'une quelconque des revendications 1 à 5, **caractérisé en ce que** l'ammoniac formé lors de la saponification est éliminé par distillation.

7. Procédé selon l'une quelconque des revendications 1 à 6, **caractérisé en ce que** les sels de l'acide glycérique de formule générale (I) formés après la saponification catalysée par des bases sont transformés en acides glycériques libres.
